# EUROPEAN PATENT APPLICATION

(11) **EP 1 944 025 A1**
(43) Date of publication of application: **16.07.2008**
(21) Application number: 07100298.4
(22) Date of filing: 09.01.2007
(51) Int. Cl.: A61K 31/365, A61P 3/04, A61P 3/06

(54) **Stable pharmaceutical compositions of orlistat**

(71) Applicant: Ranbaxy Laboratories Limited, Haryana 122001, Delhi (IN)
(72) Inventor: Deepak, Murpani, Part-I, 110024, New Dehli (IN); Keshav, Deo, 122001, Gurgaon (IN); Tarun, Vijan, Haryana, 134109, Panchkula (IN)
(74) Representative: Cronin, Brian Harold John

(57) **Abstract**

The present invention relates to stable pharmaceutical compositions of orlistat for treatment or prevention of obesity and hyperlipidemia. The pharmaceutical compositions contain Orlistat form I, which does not convert to form II at the temperatures encountered during manufacturing of an orlistat dosage form.

## Description

### Technical Field of the Invention

The present invention relates to stable pharmaceutical compositions of orlistat for treatment or prevention of obesity and hyperlipidemia. The pharmaceutical compositions contain Orlistat form I which does not convert to form II at the moderate and practical temperatures encountered during manufacturing of dosage form.

### Background of the invention

Orlistat, a tetrahydrolipstatin, is a useful pancreatic lipase-inhibiting agent and can be used for the prevention and treatment of obesity and hyperlipaemia. Chemically, it is (S)-N-formyl leucine (S)-1-[[(2S, 3S)-3-hexyl-4-oxo-2-oxetanyl]methyl]dodecyl ester and is known from U.S. Patent No. 4,598,089.

U.S. Patent No. 6,156,911 discloses a method for purification of lipstatin and also a process for production of a crystalline form of orlistat, i.e., tetrahydrolipstatin. It discloses the process for preparation of the crystalline orlistat but does not touch upon the subject of pharmaceutical compositions containing the same. This crystalline form disclosed in the '911 patent is herein after referred to as form I or form A. Another crystalline form of orlistat, having a different X-ray diffraction pattern, is marketed by Roche as Zenical^{®} capsules but has not been reported in the literature and is herein after referred to as form II or form B.

Further, our co-pending patent application 1144/DEL/2003 describes the process for preparation of crystalline forms I and II along with pharmaceutical compositions containing the same.

However, none of the prior art references disclose whether there are polymorphic conversions during the manufacturing process of the dosage form. It is one of the vital aspects of stability of these crystalline forms which is the next most sought after aspect in polymorphism other than therapeutic efficacy. It is a general question in formulation technology whether polymorphic modifications can transform during the manufacturing process. The manufacture of pharmaceutical dosage forms often requires the use of solvents, such as during granulation, which then need to be removed from the dosage form. One of the common ways of removing solvents is by the use of heated air. The active ingredient may be exposed to temperatures in the range of 40 to 60°C. These conditions can significantly contribute to polymorphic conversion.

Because polymorphic crystal forms of a specific chemical compound have different physical properties caused by different arrangements of the molecules in the crystal lattice, these different characteristics often lead to considerable differences in hygroscopicity, solubility, bioavailability, and the ease of processing into a dosage form. All these properties are of great consequence for the production of pharmaceutical formulations. Using a thermodynamically unstable polymorphic crystal form in the production of pharmaceutical compositions is sometimes the reason for unwanted changes taking place in such compositions during their manufacture and storage. These changes may include stability of the composition and solubility of the converted polymorphic crystalline form which may affect the bioavailability. Hence, it is desirable to have a crystalline form which is stable enough to handle the stresses that occur during manufacture of pharmaceutical compositions as well as during storage. Advantageously, a stable polymorphic crystalline form would not be expected to contribute to erratic bioavailability, which may otherwise appear with some batch to batch variation depending upon the amount of conversion.

Orlistat form I has a melting point of about 44°C and that of form II is about 43°C, which are quite close to each other. Despite of the fact that there is a minimal difference between the melting points of the two forms, we have found that the two forms are monotropically related to each other with form I being stable over a wider range of temperatures compared to form II. Thus, form I can be used intrepidly for the manufacture of pharmaceutical formulations without any apprehensions of being transformed into form II.

We hereby disclose stable pharmaceutical compositions containing orlistat crystalline form I, wherein the crystalline form I does not rearrange into form II over a wide range of temperature over a period of time.

### Summary of the Invention

In one general aspect there is provided a stable pharmaceutical composition comprising an effective amount of orlistat form I and a pharmaceutically acceptable carrier. The orlistat form I does not rearrange into form II and is obtained by a process that includes preparing a melt of orlistat and cooling the melt to get the orlistat form I.

Embodiments of the process may include one or more of the following features. For example, the stability may be maintained after storing the composition at 45°C to 50°C for 1 week.

The composition may be selected from tablets, capsules, granules, powder and pellets. The pharmaceutically acceptable carrier may include one or more of excipients selected from the group comprising diluents, binders, disintegrants and lubricants or glidants. The diluent may be selected from one or more of mannitol, sorbitol, xylitol, lactose, microcrystalline cellulose, magnesium carbonate, calcium carbonate, dicalcium phosphate, tribasic calcium phosphate, calcium sulphate and magnesium trisilicate. The binder may be selected from one or more of polyvinyl pyrrolidone, copovidone, hydroxypropyl cellulose and hydroxypropyl methylcellulose. The disintegrant may be selected from cross-linked carboxymethylcellulose and its sodium salt, crospovidone, sodium starch glycolate, sodium carboxymethyl cellulose, calcium carboxymethyl cellulose, low-substituted hydroxypropyl cellulose and sodium alginate. The lubricant or glidant may be selected from one or more of talc, magnesium stearate, zinc stearate, calcium stearate, sodium stearyl fumarate, stearic acid, talc and colloidal silicon dioxide.

The composition may be prepared by dry granulation, wet granulation or direct compression.

In another general aspect there is provided a process for making a pharmaceutical composition. The process includes providing orlistat form I; preparing a melt of the orlistat form I; cooling the melt to get orlistat form I; and combining the orlistat form I with a pharmaceutically acceptable carrier to form the pharmaceutical composition, wherein the orlistat form I does not rearrange into form II.

Embodiments of the process may include one or more of the following features. For example, the composition may be selected from tablets, capsules, granules, powder and pellets. The pharmaceutically acceptable carrier may include one or more excipients selected from the group comprising diluents, binders, disintegrants and lubricants or glidants.

In another general aspect there is provided a method of treating or preventing obesity and/or hyperlipaemia in a warm-blooded animal. The method includes administering a stable pharmaceutical composition comprising an effective amount of orlistat form I and a pharmaceutically acceptable carrier, wherein the orlistat form I does not rearrange into form II and wherein the orlistat form I is obtained by the process comprising: preparing a melt of orlistat; and cooling the melt to get the orlistat form I. wherein the orlistat form I does not rearrange into form II.

In another aspect, it relates to a stable pharmaceutical composition comprising an effective amount of orlistat form I and a pharmaceutically acceptable carrier, wherein the orlistat form I does not rearrange into form II upon storage at 45°C-50°C for 1 week.

### Description of the Drawings

Figures 1(a) and (b) show the X-Ray powder diffraction (XRD) pattern of capsules containing form I and form II respectively (control samples).
Figure 2(a) is a X-Ray powder diffraction pattern of capsules containing form I of orlistat kept at 45°C for 1 week.
Figure 2(b) is a X-Ray powder diffraction pattern of capsules containing form I of orlistat kept at 50°C for 1 week.
Figure 3(a) is a X-Ray powder diffraction pattern of capsules containing form II of orlistat kept at 45°C for 1 week.
Figure 3(b) is a X-Ray powder diffraction pattern of capsules containing form II of orlistat kept at 50°C for 1 week.

### Detailed Description of the Invention

The present invention relates to stable pharmaceutical compositions containing orlistat form I, a polymorphic form that has been thus far unexplored as being a practical choice as a stable polymorphic form of orlistat, that is, it does not substantially rearrange over time. This property makes orlistat form I useful for the manufacture of stable pharmaceutical compositions containing orlistat.

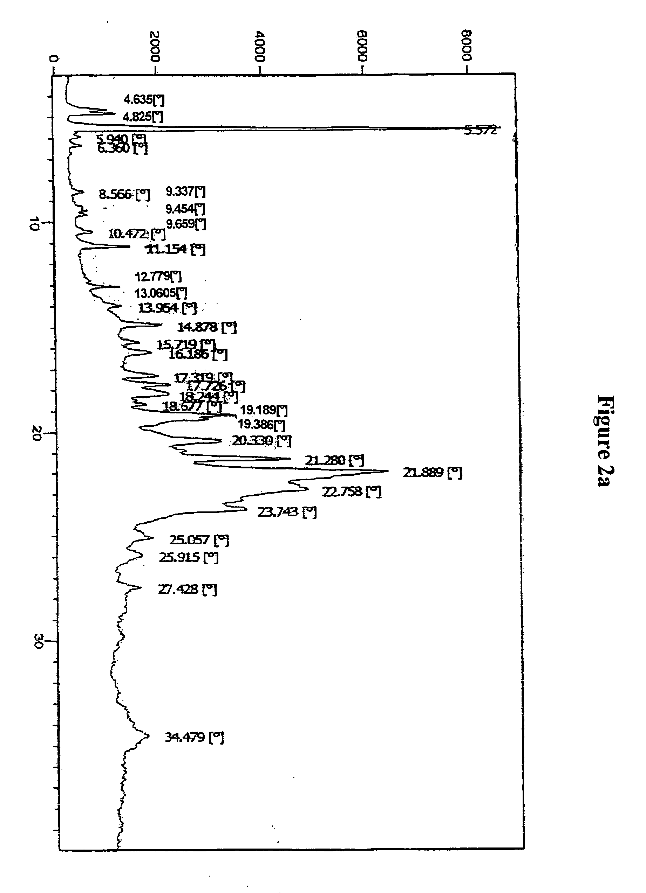

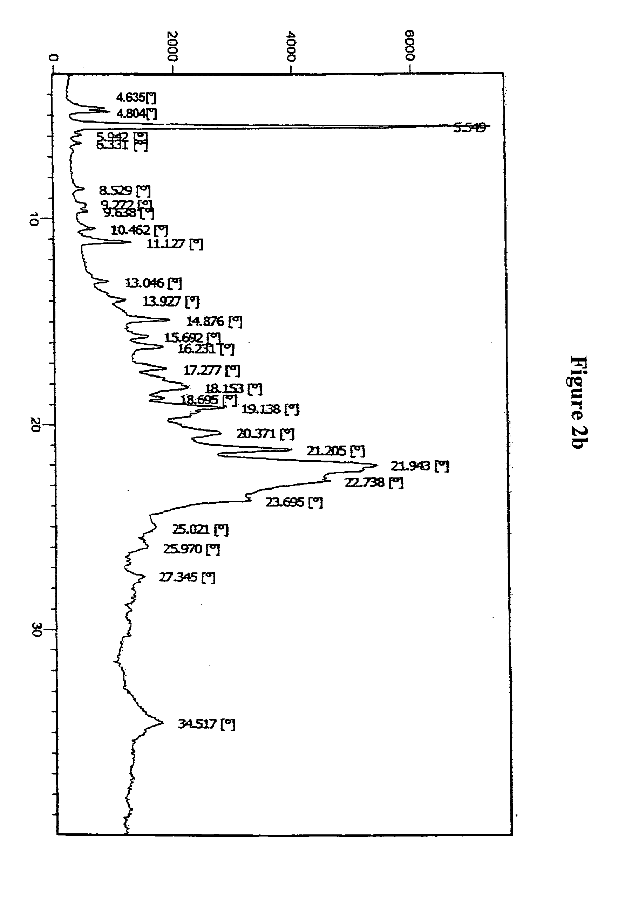

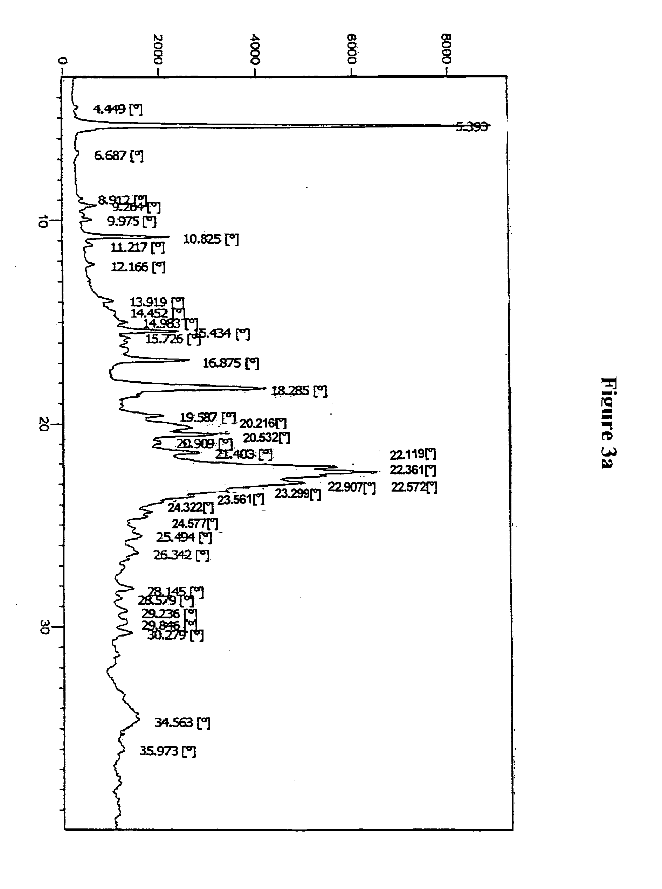

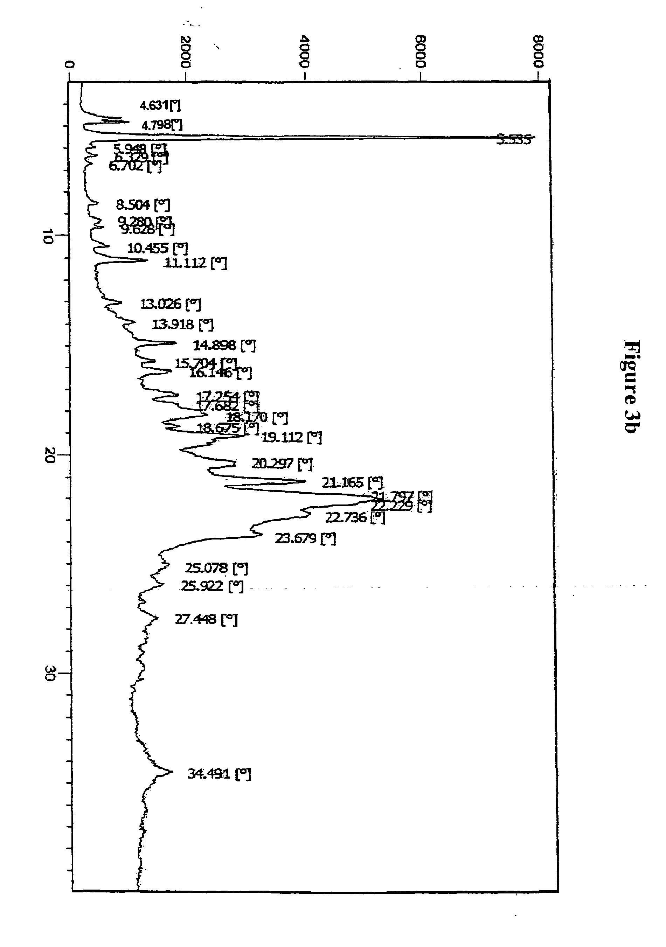

Although the two polymorphic forms of orlistat are known from the prior art, in analyzing the issue of relative stability we have surprisingly found that pharmaceutical compositions containing orlistat form I show no change in the polymorphic form of orlistat when subjected to a temperature condition of 45°C or even 50°C for a week. Figures 2(a) and (b) show the X-Ray diffraction pattern of orlistat capsules of Example 1 containing orlistat form I kept at 45°C and 50°C respectively for 1 week. In contrast, the pharmaceutical compositions containing orlistat form II show conversion to form I when kept at 45°C and 50°C for 1 week, as illustrated by the X-Ray diffraction pattern of Figures 3(a) and (b). The change was more prominent at 50°C than 45°C.

The two key areas of concern with polymorphism in pharmaceuticals are the relative bioavailability and stability of the polymorphic forms. Polymorphs of a pharmaceutical active may have different physical and solid-state chemical (e.g., reactivity) properties. The most stable polymorphic form of a drug substance is often used because it has the lowest potential for conversion from one polymorphic form to another while the metastable form may give variable bioavailability. Without wishing to be bound by any particular theory, it is believed that since orlistat form II shows a rearrangement to form I at a temperature of about 45°C-50°C, which is above the melting points of both the polymorphic forms, the two forms are monotropically related. A simple definition of monotropism says that transition occurs only in one direction from the unstable to the stable form unlike enantiotropic systems, where the polymorphic forms are in equilibrium and the transition can take place in either direction depending on the temperature. Orlistat forms I and II form a monotropic system rendering form I as stable over a wider range of temperatures thus making it suitable to be used in pharmaceutical compositions without any trepidation of physical instability or variable activity.

The present invention relates to a stable pharmaceutical composition containing orlistat form I wherein the pharmaceutical composition has the surprising and useful advantage that the active material, orlistat form I, does not rearrange into orlistat form II over a wide range of temperatures over a period of time.

The effective amount of orlistat in the pharmaceutical compositions may be varied to an amount determined by the extent of treatment required for a particular subject. The pharmaceutical composition may comprise from about 1mg to about 500mg of orlistat form 1. The preferred amount in the pharmaceutical composition is 120mg of orlistat form 1.

The pharmaceutical compositions may be, for example, tablets, capsules, granules, powder and pellets, etc. and may be prepared by any of the dry granulation, wet granulation or direct compression processes well known in the art of solid dosage forms. The pharmaceutical compositions may be designed for immediate release of the active ingredient or formulated for the controlled release of the active ingredient.

Besides the active ingredient, the dosage forms may contain diluents, binders, disintegrants, and lubricants or glidants.

Diluents may include one or more of mannitol, sorbitol, xylitol, lactose, microcrystalline cellulose, magnesium carbonate, calcium carbonate, dicalcium phosphate, tribasic calcium phosphate, calcium sulphate, magnesium trisilicate, and the like.

Binders may include one or more of polyvinylpyrrolidone, copovidone, hydroxypropyl cellulose, hydroxypropyl methylcellulose and other such materials routinely used in the art of solid dosage forms for the purpose of binding and preparation of granules.

Disintegrant may include one or more of cross-linked carboxymethylcellulose and its sodium salt, crospovidone, sodium starch glycolate, sodium carboxymethyl cellulose, calcium carboxymethyl cellulose, low-substituted hydroxypropyl cellulose and sodium alginate or any other such material routinely used in the art of solid dosage forms.

Lubricant or glidant may include one or more of talc, magnesium stearate, zinc stearate, calcium stearate, sodium stearyl fumarate, stearic acid, talc, colloidal silicon dioxide, and the like.

The following example is illustrative of the invention and is not intended to be construed as limiting the invention.

### Example 1

| **S. No.** | **Ingredients** | **Qty. Mg/cap** |
|---|---|---|
| 1. | Orlistat | 120.00 |
| 2. | Microcrystalline cellulose | 88.8 |
| 3. | Sodium starch glycolate | 10.8 |
| 4. | Sodium lauryl sulfate | 6.0 |
| 5. | Copovidone | 12.0 |
| 6. | Purified water | q.s. |
| 7. | Talc | 2.4 |

**Process:** The active ingredient (orlistat), microcrystalline cellulose, sodium starch glycolate and sodium lauryl sulfate are sifted through a suitable mesh and blended in a rapid mixer granulator followed by granulation with the binder solution of copovidone in purified water. The wet mass thus obtained is extruded, spheronized, and the resulting pellets dried in a fluid bed dryer. The dried pellets are lubricated with talc and filled into the capsule shells.

### Stability results for Orlistat capsules containing Orlistat form I:

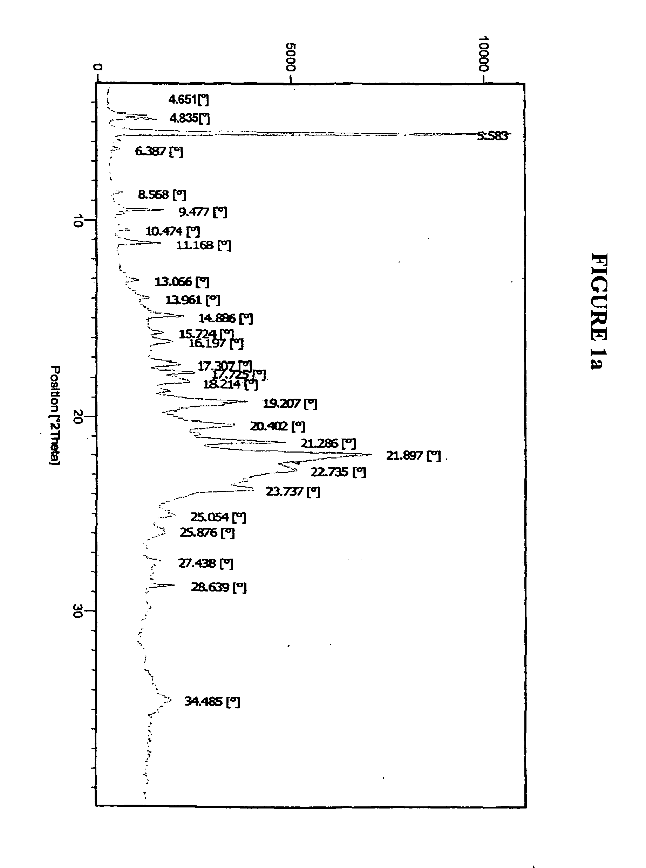

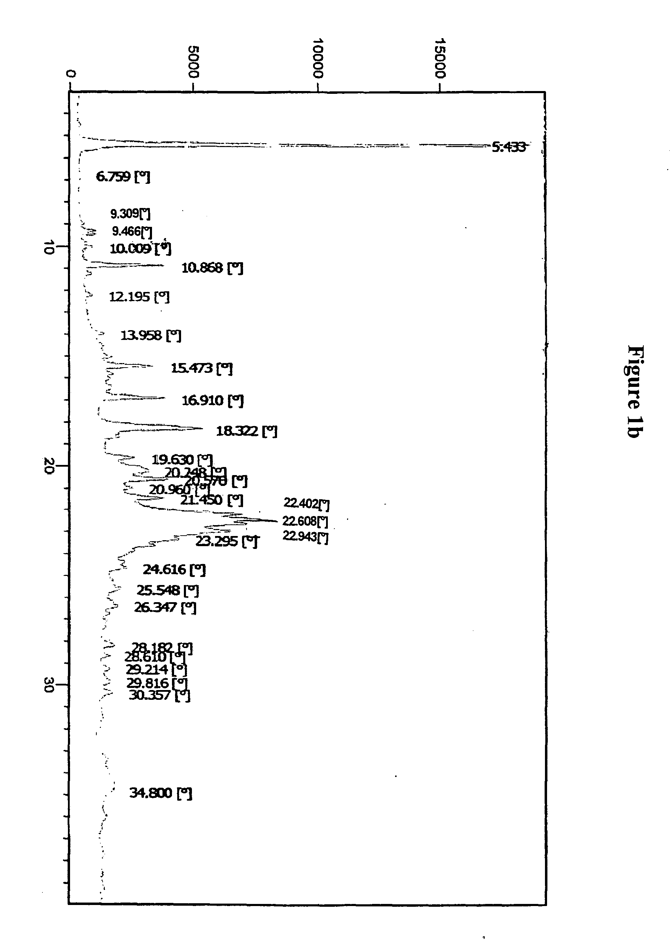

Capsules containing 120 mg of orlistat form I prepared according to the method of Example 1 were subjected to storage under the temperature conditions of 45°C and 50°C for one week. The polymorphic conversion of orlistat inside the capsule was monitored by X-ray powder diffraction (XRPD) technique. Representative X-ray powder diffraction patterns are shown in Figures 1 to 3. As evident from the figures, there is no change in the XRPD pattern of form I of orlistat in the capsules kept at either 45°C or 50°C. In contrast, capsules containing form II of orlistat show conversion into form I when kept at 45°C or 50°C for a week.

## Claims

1. A stable pharmaceutical composition comprising an effective amount of orlistat form I and a pharmaceutically acceptable carrier, wherein the orlistat form I does not rearrange into form II and wherein the orlistat form I is obtained by the process comprising: preparing a melt of orlistat; and cooling the melt to get the orlistat form I.

2. The stable pharmaceutical composition according to claim 1, wherein the stability is maintained after storing the composition at 45°C to 50°C for 1 week.

3. The stable pharmaceutical composition according to claim 1, wherein the composition is selected from tablets, capsules, granules, powder and pellets.

4. The stable pharmaceutical composition according to claim 1, wherein the pharmaceutically acceptable carrier comprises one or more of excipients selected from the group comprising diluents, binders, disintegrants and lubricants or glidants.

5. The stable pharmaceutical composition according to claim 4 wherein the diluent is selected from one or more of mannitol, sorbitol, xylitol, lactose, microcrystalline cellulose, magnesium carbonate, calcium carbonate, dicalcium phosphate, tribasic calcium phosphate, calcium sulphate and magnesium trisilicate.

6. The stable pharmaceutical composition according to claim 4 wherein the binder is selected from one or more of polyvinyl pyrrolidone, copovidone, hydroxypropyl cellulose and hydroxypropyl methylcellulose.

7. The stable pharmaceutical composition according to claim 4 wherein the disintegrant is selected from cross-linked carboxymethylcellulose and its sodium salt, crospovidone, sodium starch glycolate, sodium carboxymethyl cellulose, calcium carboxymethyl cellulose, low-substituted hydroxypropyl cellulose and sodium alginate.

8. The stable pharmaceutical composition according to claim 4 wherein the lubricant or glidant is selected from one or more of talc, magnesium stearate, zinc stearate, calcium stearate, sodium stearyl fumarate, stearic acid, talc and colloidal silicon dioxide.

9. The stable pharmaceutical composition according to claim 1, wherein the composition is prepared by dry granulation, wet granulation or direct compression.

10. A process for making a pharmaceutical composition, the process comprising: providing orlistat,
preparing a melt of the orlistat;
cooling the melt to get orlistat form I; and
combining the orlistat form I with a pharmaceutically acceptable carrier to form the pharmaceutical composition, wherein the orlistat form I does not rearrange into form II.

11. The process according to claim 10, wherein the composition is selected from tablets, capsules, granules, powder and pellets.

12. The process according to claim 10, wherein the pharmaceutically acceptable carrier comprises one or more of excipients selected from the group comprising diluents, binders, disintegrants and lubricants or glidants.

13. A method of treating or preventing obesity and/or hyperlipaemia in a warm-blooded animal comprising administering a stable pharmaceutical composition comprising an effective amount of orlistat form I and a pharmaceutically acceptable carrier, wherein the orlistat form I does not rearrange into form II and wherein the orlistat form I is obtained by the process comprising: preparing a melt of orlistat; and cooling the melt to get the orlistat form I. wherein the orlistat form I does not rearrange into form II.
